# FASCICULE DE BREVET EUROPEEN

(11) **EP 4 417 973 B1**
(45) Date de publication et mention de la délivrance du brevet: **10.12.2025**
(21) Numéro de dépôt: 24157842.6
(22) Date de dépôt: 15.02.2024
(51) Int. Cl.: G01N 33/28, B03C 1/28

(54) **SONDE DESTINÉE À VÉRIFIER LA PRÉSENCE DE PARTICULES PARAMAGNÉTIQUES DANS UN RÉSERVOIR**
SONDE ZUR ÜBERPRÜFUNG DER ANWESENHEIT PARAMAGNETISCHER TEILCHEN IN EINEM BEHÄLTER
PROBE FOR CHECKING THE PRESENCE OF PARAMAGNETIC PARTICLES IN A RESERVOIR

(30) Priorité: 17.02.2023 FR 2301482
(43) Date de publication de la demande: 21.08.2024
(73) Titulaire: AIRBUS OPERATIONS, 31060 Toulouse Cedex 9 (FR)
(72) Inventeur: PETIT, Patrick, 31060 TOULOUSE (FR); MILLET, Gérard, 31060 TOULOUSE (FR); LOPEZ, Thierry, 31060 TOULOUSE (FR); MONTEIL, Alexis, 31060 TOULOUSE (FR)
(74) Mandataire: Cabinet Le Guen Maillet

(56) Documents cités:
- CN-A- 113 899 662
- US-A- 2 830 261
- US-A- 4 537 071
- US-B1- 9 632 072
- US-B2- 10 539 054

## Description

### DOMAINE TECHNIQUE

La présente invention concerne une sonde destinée à vérifier la présence de particules paramagnétiques dans un réservoir contenant un fluide potentiellement chargé de ces particules paramagnétiques et ceci sans vider ledit réservoir. La présente invention concerne également un ensemble comportant un réservoir contenant le fluide et une telle sonde.

### ÉTAT DE LA TECHNIQUE ANTÉRIEURE

En mécanique, de nombreux mécanismes mobiles, comme les boîtes de vitesse, les générateurs électriques, sont logés dans un réservoir contenant un fluide, typiquement de l'huile, pour assurer la lubrification du mécanisme. Avec l'usure des pièces mécaniques constituant le mécanisme, des particules arrachées à ces pièces se dispersent dans le réservoir. Il est alors nécessaire de purger le réservoir de son huile pour la remplacer par une huile neuve.

Dans le cadre de la maintenance d'un aéronef, ce remplacement de l'huile peut être relativement long et coûteux du fait de l'immobilisation de l'aéronef. Il est donc souhaitable de trouver un arrangement qui limite les besoins de vidange et qui, en outre, permet de connaître l'état d'usure des pièces mécaniques sans vidanger l'huile pour optimiser les opérations de maintenance. US 9 632 072 divulgue une sonde pour vérifier la présence de particules paramagnétique .

### EXPOSÉ DE L'INVENTION

Un objet de la présente invention est de proposer une sonde pour un réservoir contenant un fluide potentiellement chargé de particules paramagnétiques, où la sonde est destinée à vérifier la présence de ces particules paramagnétiques sans vider entièrement ledit réservoir. À cet effet, est proposée une sonde destinée à être utilisée avec un réservoir présentant une paroi percée d'un orifice et où le réservoir est destiné à contenir un fluide chargé de particules paramagnétiques, ladite sonde comportant :
- un aimant,
- un système de déplacement destiné à être arrangé au niveau de l'orifice et agencé pour déplacer l'aimant d'une position de captage dans laquelle l'aimant baigne dans le fluide à une position de vérification dans laquelle l'aimant est hors du réservoir, où le système de déplacement est agencé pour déplacer l'aimant à travers l'orifice de l'intérieur vers l'extérieur du réservoir et inversement, et
- des moyens d'étanchéité destinés à assurer l'étanchéité de l'orifice lors du passage de la position de captage à la position de vérification et inversement,
la sonde étant caractérisée en ce que le système de déplacement prend la forme d'une vis avec une tige filetée et une tête, en ce que l'orifice est un trou taraudé dans lequel la tige filetée se visse, en ce que la tige filetée présente un évidement dans lequel l'aimant est fixé et en ce que la position de l'évidement le long de la tige filetée est telle que l'aimant baigne dans le fluide en position de captage et est en dehors du réservoir en position de vérification.

Avec un tel arrangement, il est possible de vérifier la présence de particules paramagnétiques sans vider entièrement le réservoir par retrait partiel de la vis, ce qui permet également d'évaluer l'usure des pièces mécaniques d'où proviennent les particules paramagnétiques. Avantageusement, les moyens d'étanchéité sont réalisés par le filetage de la tige filetée et le taraudage de l'orifice.

Avantageusement, la vis est réalisée dans un matériau amagnétique.

Avantageusement, l'évidement prend la forme d'au moins un tunnel débouchant à chaque extrémité au niveau du filetage de la tige filetée, le ou chaque tunnel présente une saignée s'étendant le long dudit tunnel, l'aimant est logé dans la saignée à distance du filetage, et pour chaque partie d'une saignée s'étendant entre l'aimant et le filetage de la tige filetée, un élément réalisé dans un matériau amagnétique est logé dans ladite partie.

L'invention propose également un ensemble comportant un réservoir présentant une paroi percée d'un orifice et où le réservoir est destiné à contenir un fluide chargé de particules paramagnétiques et une sonde selon l'une des variantes précédentes, où le système de déplacement est arrangé au niveau de l'orifice et agencé pour déplacer l'aimant d'une position de captage dans laquelle l'aimant baigne dans le fluide à une position de vérification dans laquelle l'aimant est hors du réservoir, et où les moyens d'étanchéité sont arrangés pour assurer l'étanchéité de l'orifice lors du passage de la position de captage à la position de vérification et inversement.

### BRÈVE DESCRIPTION DES DESSINS

Les caractéristiques de l'invention mentionnées ci-dessus, ainsi que d'autres, apparaîtront plus clairement à la lecture de la description suivante d'un exemple de réalisation, ladite description étant faite en relation avec les dessins joints, parmi lesquels :
[Fig. 1] est une vue en coupe d'un ensemble avec une sonde selon l'invention dans une position de captage de la sonde,
[Fig. 2] est une vue en coupe de l'ensemble de la Fig. 1 dans une position de vérification de la sonde,
[Fig. 3] est une vue en perspective de la sonde selon l'invention,
[Fig. 4] est une vue en coupe d'un ensemble avec une sonde dans une position de captage de la sonde,
[Fig. 5] est une vue en coupe de l'ensemble de la Fig. 4 dans une position d'isolation de la sonde,
[Fig. 6] est une vue en coupe de l'ensemble de la Fig. 4 dans une position de vérification de la sonde, et
[Fig. 7] est une vue selon la flèche VII de la Fig. 4 dans une position de la sonde intermédiaire entre la position de captage et la position d'isolation.

### EXPOSÉ DÉTAILLÉ DE MODES DE RÉALISATION

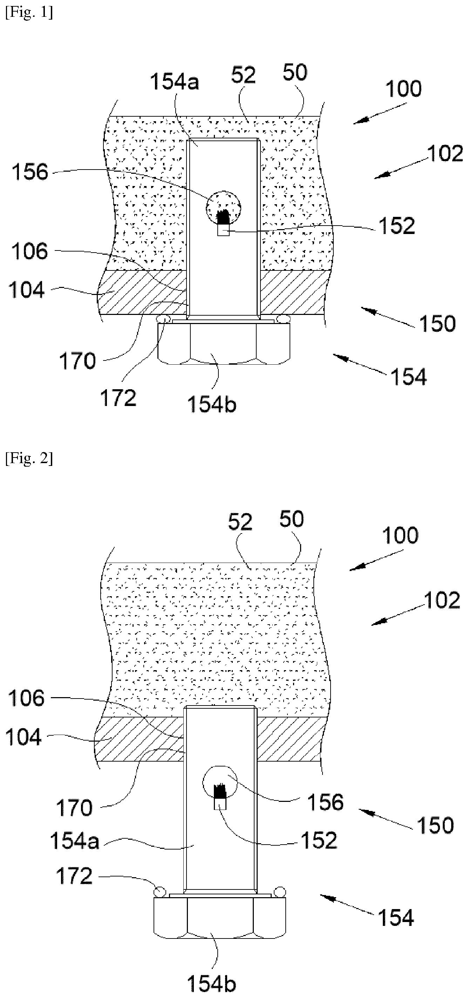

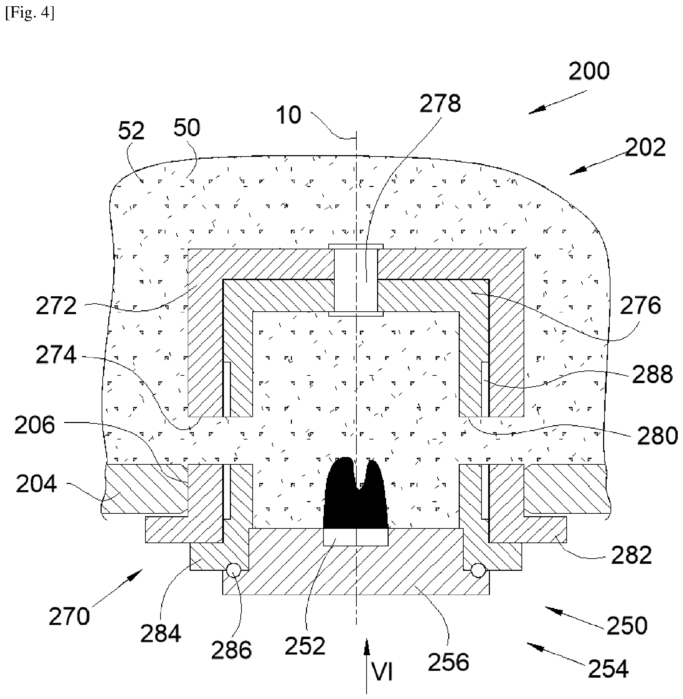

Les Figs. 1 à 3 montrent un ensemble 100 avec une sonde 150 selon un mode de réalisation de l'invention, et les Figs. 4 à 7 montrent un ensemble 200 avec une sonde 250 selon un mode de réalisation non revendiqué. Chacun de ces modes de réalisation permet de vérifier la présence de particules paramagnétiques dans un réservoir sans avoir à vider entièrement ledit réservoir et également d'évaluer l'usure des pièces mécaniques d'où proviennent les particules paramagnétiques. Comme cela est présenté ci-dessous, la sonde 150, 200 constitue un bouchon pour un réservoir 102, 202 et ce bouchon comporte des moyens pour réaliser un sondage à l'intérieur du réservoir 102, 202.

L'ensemble 100, 200 comporte un réservoir 102, 202 présentant une paroi 104, 204 percée d'un orifice 106, 206 et où le réservoir 102, 202 est destiné à contenir un fluide 50. Le fluide 50 est par exemple de l'huile qui assure la lubrification des pièces métalliques en mouvement qui sont logées dans le réservoir, comme par exemple les engrenages d'une boîte de vitesse, et qui baignent dans le fluide 50. Par usure, les pièces métalliques libèrent des particules paramagnétiques 52.

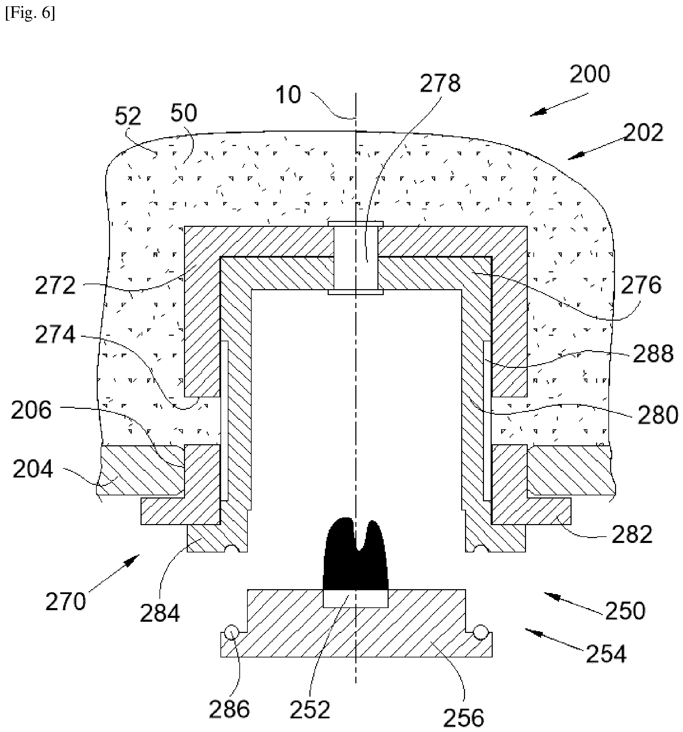

L'ensemble 100, 200 comporte également la sonde 150, 250 qui porte un aimant 152, 252. La sonde 150, 250 comporte également un système de déplacement 154, 254 qui est arrangé au niveau de l'orifice 106, 206 et qui est agencé pour déplacer l'aimant 152, 252 d'une position de captage (Fig. 1, Fig. 4) dans laquelle l'aimant 152, 252 baigne dans le fluide 50 de manière à ce qu'au moins une partie des particules paramagnétiques 52 se fixent audit aimant 152, 252, à une position de vérification (Fig. 2, Fig. 6) dans laquelle l'aimant 152, 252 ne baigne pas dans le fluide 50 et est hors du réservoir 102, 202 pour être visible avec les particules paramagnétiques 52 qui y sont fixées depuis l'extérieur du réservoir 100, 200, et inversement.

La sonde 150, 250 comporte également des moyens d'étanchéité 170, 270 qui sont arrangés pour assurer l'étanchéité de l'orifice 106, 206 lors du passage de la position de captage à la position de vérification et inversement. Ainsi, le passage de la position de captage à la position de vérification et inversement est tel que le réservoir 102, 202 ne se vide pas entièrement du fluide 50 et plus particulièrement, il se vide de manière très limitée dans les deux modes de réalisation comme cela est expliqué ci-dessous.

Les moyens d'étanchéité 170, 270 assurent également l'étanchéité de l'orifice 106, 206 vis-à-vis du réservoir 102, 202 pour toutes positions entre la position de captage et la position de vérification et dans la position de captage et la position de vérification.

Ainsi, il est possible de connaître l'état de contamination du fluide 50 sans être obligé de vidanger le réservoir 102, 202 d'où un gain de temps et une réduction de la consommation de fluide 50.

Dans les deux modes de réalisation présentés sur les Figs. 1 et 4, l'orifice 106, 206 est au niveau d'une paroi inférieure du réservoir 102, 202 car il peut également servir d'orifice de vidange pour vider entièrement le fluide 50 en cas de besoin, mais l'orifice 106, 206 peut également être réalisé au niveau d'une autre paroi du réservoir 102, 202, tant que l'aimant 152, 252 baigne dans le fluide 50 en position de captage.

Dans le mode de réalisation de l'invention, le système de déplacement 154 est agencé pour déplacer l'aimant 152 à travers l'orifice 106 de l'intérieur vers l'extérieur du réservoir 102 et inversement, et les moyens d'étanchéité 170 sont arrangés pour assurer l'étanchéité au niveau de l'orifice 106 lorsque l'aimant 152 le traverse. La position de captage correspond à l'aimant 152 à l'intérieur du réservoir 102 et la position de vérification correspond à l'aimant 152 à l'extérieur du réservoir 102.

Le système de déplacement 154 prend la forme d'une vis avec une tige filetée 154a et une tête 154b et l'orifice 106 est un trou taraudé dans lequel la tige filetée 154a se visse. La tête 154b reste à l'extérieur du réservoir 102.

La tige filetée 154a présente au moins un évidement 156 dans chacun duquel ou desquels un aimant 152 est fixé à la tige filetée 154a. Dans le mode de réalisation de l'invention présenté ici, l'évidement 156 est traversant de part en part de la tige filetée 154a.

La position de l'évidement 156 le long de la tige filetée 154a est telle que l'aimant 152 baigne dans le fluide 50 en position de captage et est en dehors du réservoir 102 en position de vérification. Le passage de la position de captage à la position de vérification consiste à dévisser la vis, et inversement le passage de la position de vérification à la position de captage consiste à visser la vis.

Ainsi le fluide 50 se répand dans l'évidement 156 et au moins une partie des particules paramagnétiques 52 se fixent à l'aimant 152.

En position de vérification, la vis n'est pas entièrement dévissée et la tige filetée 154a reste en prise dans l'orifice 106 pour éviter les fuites de fluide 50. Seul le fluide 50 présent dans l'évidement 156 est retiré du réservoir 102 ce qui représente une quantité très faible qui peut être facilement compensée par une mise à niveau ultérieure à moindre coût par rapport à l'état de la technique.

Lorsque l'orifice 106 constitue également l'orifice de vidange, la tige filetée 154 est entièrement retirée pour laisser le fluide 50 s'écouler.

Les moyens d'étanchéité 170 sont réalisés par le filetage de la tige filetée 154a et le taraudage de l'orifice 106 qui coopèrent pour assurer l'étanchéité. Préférentiellement, pour une meilleure étanchéité en position de vérification, la position de l'évidement 156 est telle qu'en position de vérification, la tige filetée 154a reste en prise avec l'orifice 106 sur toute la profondeur de ce dernier.

Dans le mode de réalisation de l'invention présenté à la Fig. 1, l'étanchéité est complétée par un joint 172, par exemple du type joint torique, qui est disposé entre la tête 154b et la paroi 104 et autour de l'orifice 106.

Selon un mode de réalisation particulier, la vis est réalisée dans un matériau amagnétique, ainsi, les particules paramagnétiques 52 ne viennent pas se fixer sur la tige filetée 154a et en particulier elles ne se fixent pas sur le filetage.

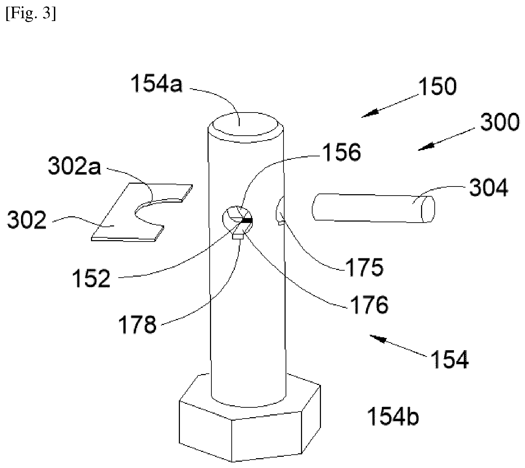

La Fig. 3 montre un mode de réalisation particulier, où la vis peut être réalisée dans un matériau paramagnétique, et pour éviter la magnétisation de la tige filetée 154a du fait de la présence de l'aimant 152 qui y est fixé, des éléments 176 réalisés dans un matériau amagnétique sont disposés et fixés entre l'aimant 152 et le filetage de la tige filetée 154a. Bien sûr, pour pouvoir capter les particules paramagnétiques 52, au moins une face de l'aimant 152 reste libre, c'est-à-dire qu'elle n'est en contact ni avec la tige filetée 154a ni avec un élément 176.

L'évidement 156 prend la forme d'au moins un tunnel 175 débouchant à chaque extrémité au niveau du filetage de la tige filetée 154a. ici, il y a deux tunnels 175 en croix. Chaque tunnel 175 présente une saignée 178 s'étendant le long dudit tunnel 175 et l'aimant 152 est logé dans la saignée 178 à distance du filetage, ici l'aimant 152 est logé à l'intersection des deux saignées 178. Pour chaque partie d'une saignée 178 s'étendant entre l'aimant 152 et le filetage de la tige filetée 154a, un élément 176 réalisé dans un matériau amagnétique, ici une barrette, est logée dans ladite partie dans le prolongement de l'aimant 152 jusqu'à l'extrémité du tunnel 175 correspondant et débouchant au niveau du filetage de la tige filetée 154a.

Ici, l'aimant 152 présente une face libre qui affleure la surface des éléments 176.

La Fig. 3 montre également un outil 300 qui permet de récupérer les particules paramagnétiques 52 qui sont liées à l'aimant 152. L'outil 300 comporte un réceptacle 302 présentant une découpe 302a dont la forme est complémentaire à la forme de la tige filetée 154a, c'est-à-dire une découpe en arc de cercle. L'outil 300 comporte également un piston 304 dont la forme est adaptée à la forme de l'évidement 156.

La récupération des particules paramagnétiques 52 consiste à placer le réceptacle 302 contre la tige filetée 154a à une extrémité de l'évidement 156 et à introduire le piston 304 par l'autre extrémité de l'évidement 156 en le déplaçant vers le réceptacle 302 en frottant contre l'aimant 152 pour récupérer les particules paramagnétiques 52 qui sont alors récupérées dans le réceptacle.

Il est alors possible d'analyser la quantité de particules paramagnétiques 52 et en fonction de cette quantité de décider de faire une vidange complète ou non du réservoir 102.

Dans le mode de réalisation non revendiqué, le système de déplacement 254 est agencé pour déplacer l'aimant 252 entre la position de captage (Fig. 4) dans laquelle l'aimant 252 est en contact avec le fluide 50 à travers l'orifice 206 et la position de vérification (Fig. 6) dans laquelle l'aimant 252 n'est pas en contact avec le fluide 50 et inversement.

Dans le mode de réalisation non revendiqué, les moyens d'étanchéité 270 prennent la forme d'un système de double cloche monté au niveau de l'orifice 206.

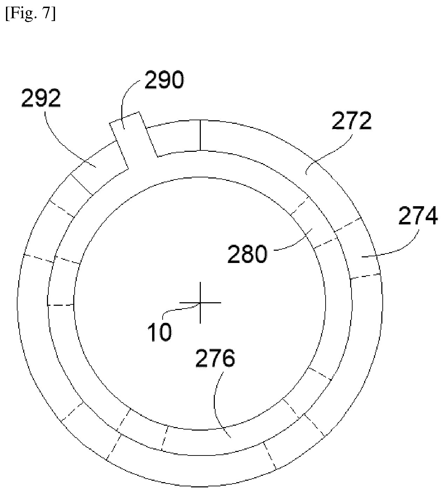

Les moyens d'étanchéité 270 comportent une cloche extérieure qui prend la forme d'un premier cylindre borgne 272 qui présente une extrémité ouverte qui est montée fixe dans l'orifice 206 par exemple par vissage et une extrémité borgne plongeant dans le fluide 50. La partie de la paroi cylindrique du premier cylindre borgne 272 qui est plongée dans le fluide 50 est percée de premiers passages 274 par lesquels le fluide 50 peut passer. Comme le montre la Fig. 7, il y a plusieurs premiers passages 274 distants les uns des autres et séparés par des parties pleines de la paroi cylindrique du premier cylindre borgne 272. Dans le mode de réalisation non revendiqué présenté aux Figs. 4 à 6, l'extrémité ouverte du premier cylindre borgne 272 présente un premier épaulement 282 qui vient en appui contre une face extérieure de la paroi 204 du réservoir 202.

Les moyens d'étanchéité 270 comportent une cloche intérieure qui prend la forme d'un deuxième cylindre borgne 276 qui est monté mobile en rotation à l'intérieur du premier cylindre borgne 272. Le deuxième cylindre borgne 276 présente une extrémité ouverte qui est disposée au niveau de l'extrémité ouverte du premier cylindre borgne 272 et une extrémité borgne disposée contre l'extrémité borgne du premier cylindre borgne 272. Dans le mode de réalisation non revendiqué présenté aux Figs. 4 à 6, l'extrémité ouverte du deuxième cylindre borgne 276 présente un deuxième épaulement 284 qui vient en appui contre la paroi cylindrique du premier cylindre borgne 272 au niveau de son extrémité ouverte.

L'axe de chaque cylindre borgne 272, 276 est ici coaxial avec un même axe 10 qui est perpendiculaire au plan dans lequel l'orifice 206 est inscrit et qui constitue l'axe de rotation du deuxième cylindre borgne 276.

La liaison pivot entre le premier cylindre borgne 272 et le deuxième cylindre borgne 276 est réalisée ici par un axe 278 traversant les deux extrémités borgnes et coaxial avec l'axe 10. La paroi cylindrique du deuxième cylindre borgne 276 est également percée de deuxièmes passages 280 par lesquels le fluide 50 peut passer. Comme le montre la Fig. 7, il y a plusieurs deuxièmes passages 280 distants les uns des autres et séparés par des parties pleines de la paroi cylindrique du deuxième cylindre borgne 276.

Les premiers passages 274 et les deuxièmes passages 280 sont disposés de manière à ce qu'en positon de captage (Fig. 4), un deuxième passage 280 est en face d'un premier passage 274 pour créer un couloir par lequel le fluide 50 et les particules paramagnétiques 52 passent pour rejoindre l'intérieur du deuxième cylindre borgne 276 et de manière à ce qu'en position de vérification (Fig. 6), chaque deuxième passage 280 se trouve en face d'une partie pleine de la paroi cylindrique du premier cylindre borgne 272 et chaque premier passage 274 se trouve en face d'une partie pleine de la paroi cylindrique du deuxième cylindre borgne 276 afin de fermer le couloir et arrêter l'écoulement du fluide 50 vers l'intérieur du deuxième cylindre borgne 276.

Dans le mode de réalisation non revendiqué, le système de déplacement 254 prend la forme d'un bouchon 256 qui est vissé au deuxième cylindre borgne 276 au niveau de son extrémité ouverte et où l'aimant 252 est fixé à une face du bouchon 256 orientée vers l'intérieur du deuxième cylindre borgne 276. Le bouchon 256 peut être retiré pour donner accès à l'aimant 252.

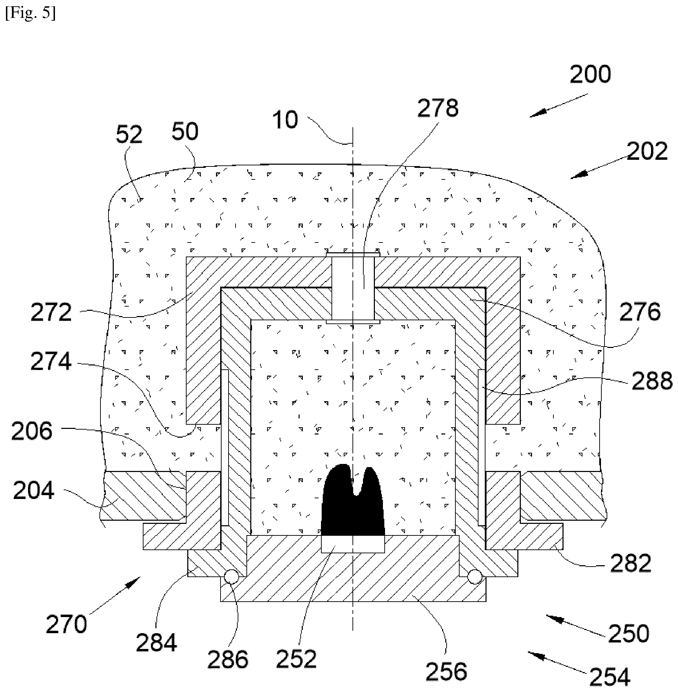

La Fig. 5 montre une position intermédiaire entre la position de captage et la position de vérification. La position intermédiaire correspond à une position d'isolation, où l'écoulement du fluide 50 à travers les passages 274 et 280 est arrêté et où le bouchon 256 n'a pas encore été retiré.

Ainsi, en partant de la position de captage (Fig. 4), le fluide 50 et les particules paramagnétiques 52 pénètrent à l'intérieur du deuxième cylindre borgne 276 et les particules paramagnétiques 52 se fixent à l'aimant 252. En passant à la position d'isolation (Fig. 5), le fluide 50 ne peut plus pénétrer dans le deuxième cylindre borgne 276. Il est alors possible de passer à la position de vérification (Fig. 6) en retirant le bouchon 256 et il est alors possible d'analyser la quantité de particules paramagnétiques 52 et en fonction de cette quantité, de décider de faire une vidange complète ou non du réservoir 202. Le passage d'une position à une autre se fait par rotation du deuxième cylindre borgne 276 par rapport au premier cylindre borgne 272. Le cheminement inverse permet de revenir à la position de captage.

Dans ce mode de réalisation, seul le fluide 50 présent dans le deuxième cylindre borgne 276 est retiré du réservoir 202 ce qui représente une quantité très faible qui peut être facilement compensée par une mise à niveau ultérieure à moindre coût par rapport à l'état de la technique.

Dans le mode de réalisation non revendiqué présenté sur les Figs. 4 à 6, un joint 286, par exemple du type joint torique, est disposé entre le bouchon 256 et le deuxième cylindre borgne 276.

Dans le mode de réalisation non revendiqué présenté sur les Figs. 4 à 6, pour chaque premier passage 274, le deuxième cylindre borgne 276 porte un joint 288 qui est fixé à la partie pleine de la paroi cylindrique du deuxième cylindre borgne 276 qui vient en face du premier passage 274 en position de vérification. Ce joint 288 prend la forme d'un élément souple, par exemple du type silicone ou caoutchouc, qui est comprimé entre le premier cylindre borgne 272 et le deuxième cylindre borgne 276. Il peut y avoir un joint 288 par premier passage 274 fixé au deuxième cylindre borgne 276 ou un seul joint 288 de forme cylindrique enfilé autour du deuxième cylindre borgne 276 et découpé en face de chaque deuxième passage 280.

La Fig. 7 montre une position intermédiaire entre la position de captage et la position d'isolation puisque les premiers passages 274 et les deuxième passages 280 sont partiellement alignés.

Dans ce mode de réalisation, le deuxième cylindre borgne 276 présente une poignée 290 qui est montée mobile dans une échancrure 292 formée dans le premier cylindre borgne 272.

L'échancrure 292 s'étend radialement entre deux bornes matérialisant la position de captage et la position d'isolation.

## Revendications

1. Sonde (150) destinée à être utilisée avec un réservoir (102) présentant une paroi (104) percée d'un orifice (106) et où le réservoir (102) est destiné à contenir un fluide (50) chargé de particules paramagnétiques (52), ladite sonde (150) comportant :
- un aimant (152),
- un système de déplacement (154) destiné à être arrangé au niveau de l'orifice (106) et agencé pour déplacer l'aimant (152) d'une position de captage dans laquelle l'aimant (152) baigne dans le fluide (50) à une position de vérification dans laquelle l'aimant (152) est hors du réservoir (102), où le système de déplacement (154) est agencé pour déplacer l'aimant (152) à travers l'orifice (106) de l'intérieur vers l'extérieur du réservoir (102) et inversement et
- des moyens d'étanchéité (170) destinés à assurer l'étanchéité de l'orifice (106) lors du passage de la position de captage à la position de vérification et inversement, la sonde (150) étant **caractérisée en ce que** le système de déplacement (154) prend la forme d'une vis avec une tige filetée (154a) et une tête (154b), **en ce que** l'orifice (106) est un trou taraudé dans lequel la tige filetée (154a) se visse, **en ce que** la tige filetée (154a) présente un évidement (156) dans lequel l'aimant (152) est fixé et **en ce que** la position de l'évidement (156) le long de la tige filetée (154a) est telle que l'aimant (152) baigne dans le fluide (50) en position de captage et est en dehors du réservoir (102) en position de vérification.

2. Sonde (150) selon la revendication 1, **caractérisée en ce que** les moyens d'étanchéité (170) sont réalisés par le filetage de la tige filetée (154a) et le taraudage de l'orifice (106).

3. Sonde (150) selon l'une des revendications 1 ou 2, **caractérisée en ce que** la vis est réalisée dans un matériau amagnétique.

4. Sonde (150) selon l'une des revendications 1 à 3, **caractérisée en ce que** l'évidement (156) prend la forme d'au moins un tunnel (175) débouchant à chaque extrémité au niveau du filetage de la tige filetée (154a), **en ce que** le ou chaque tunnel (175) présente une saignée (178) s'étendant le long dudit tunnel (175), **en ce que** l'aimant (152) est logé dans la saignée (178) à distance du filetage, et **en ce que** pour chaque partie d'une saignée (178) s'étendant entre l'aimant (152) et le filetage de la tige filetée (154a), un élément (176) réalisé dans un matériau amagnétique est logé dans ladite partie.

5. Ensemble (100) comportant un réservoir (102) présentant une paroi (104) percée d'un orifice (106) et où le réservoir (102) est destiné à contenir un fluide (50) chargé de particules paramagnétiques (52) et une sonde (150) selon l'une des revendications précédentes, où le système de déplacement (154) est arrangé au niveau de l'orifice (106) et agencé pour déplacer l'aimant (152) d'une position de captage dans laquelle l'aimant (152) baigne dans le fluide (50) à une position de vérification dans laquelle l'aimant (152) est hors du réservoir (102), et où les moyens d'étanchéité (170) sont arrangés pour assurer l'étanchéité de l'orifice (106) lors du passage de la position de captage à la position de vérification et inversement.

## Patentansprüche

1. Sonde (150), die dazu bestimmt ist, mit einem Behälter (102) verwendet zu werden, der eine Wand (104) aufweist, die eine Öffnung (106) besitzt, und wobei der Behälter (102) dazu bestimmt ist, eine Flüssigkeit (50) zu enthalten, die mit paramagnetischen Partikeln (52) belastet ist, wobei die Sonde (150) umfasst:
- einen Magneten (152),
- ein Verlagerungssystem (154), das dazu bestimmt ist, an der Öffnung (106) angeordnet zu werden, und dazu eingerichtet ist, den Magneten (152) aus einer Erfassungsposition, in welcher der Magnet (152) in die Flüssigkeit (50) eintaucht, in eine Überprüfungsposition, in welcher der Magnet (152) außerhalb des Behälters (102) ist, zu verlagern, wobei das Verlagerungssystem (154) dazu eingerichtet ist, den Magneten (152) durch die Öffnung (106) hindurch vom Innenraum zum Außenraum des Behälters (102) und umgekehrt zu verlagern, und
- Dichtungsmittel (170), die dazu bestimmt sind, die Dichtheit der Öffnung (106) beim Übergang aus der Erfassungsposition in die Überprüfungsposition und umgekehrt zu gewährleisten, wobei die Sonde (150) **dadurch gekennzeichnet ist, dass** das Verlagerungssystem (154) die Form einer Schraube mit einem Außengewindeschaft (154a) und einem Kopf (154b) annimmt, dass die Öffnung (106) ein Innengewindeloch ist, in das der Außengewindeschaft (154a) geschraubt wird, dass der Außengewindeschaft (154a) eine Ausnehmung (156) aufweist, in welcher der Magnet (152) fixiert ist, und dass die Position der Ausnehmung (156) entlang des Außengewindeschafts (154a) dergestalt ist, dass der Magnet (152) in der Erfassungsposition in die Flüssigkeit (50) eintaucht und in der Überprüfungsposition außerhalb des Behälters (102) ist.

2. Sonde (150) nach Anspruch 1, **dadurch gekennzeichnet, dass** die Dichtungsmittel (170) durch das Außengewinde des Außengewindeschafts (154a) und das Innengewinde der Öffnung (106) ausgeführt sind.

3. Sonde (150) nach einem der Ansprüche 1 oder 2, **dadurch gekennzeichnet, dass** die Schraube aus einem nichtmagnetischen Material ausgeführt ist.

4. Sonde (150) nach einem der Ansprüche 1 bis 3, **dadurch gekennzeichnet, dass** die Ausnehmung (156) die Form mindestens eines Tunnels (175) annimmt, der an jedem Ende an dem Außengewinde des Außengewindeschafts (154a) mündet, dass der oder jeder Tunnel (175) eine Vertiefung (178) aufweist, die sich entlang des Tunnels (175) erstreckt, dass der Magnet (152) beabstandet von dem Außengewinde in der Vertiefung (178) aufgenommen ist und dass bei jedem Abschnitt einer Vertiefung (178), der sich zwischen dem Magneten (152) und dem Außengewindeschaft (154a) erstreckt, ein aus einem nichtmagnetischen Material ausgeführtes Element (176) in dem Abschnitt aufgenommen ist.

5. Anordnung (100), umfassend einen Behälter (102), der eine Wand (104) aufweist, die eine Öffnung (106) besitzt, und wobei der Behälter (102) dazu bestimmt ist, eine Flüssigkeit (50) zu enthalten, die mit paramagnetischen Partikeln (52) belastet ist, und eine Sonde (150) nach einem der vorhergehenden Ansprüche, wobei das Verlagerungssystem (154) an der Öffnung (106) angeordnet ist und dazu eingerichtet ist, den Magneten (152) aus einer Erfassungsposition, in welcher der Magnet (152) in die Flüssigkeit (50) eintaucht, in eine Überprüfungsposition zu verlagern, in welcher der Magnet (152) außerhalb des Behälters (102) ist, und wobei die Dichtungsmittel (170) angeordnet sind, um die Dichtheit der Öffnung (106) beim Übergang aus der Erfassungsposition in die Überprüfungsposition und umgekehrt zu gewährleisten.

## Claims

1. Probe (150) intended to be used with a tank (102) having a wall (104) pierced by an orifice (106) and in which the tank (102) is intended to contain a fluid (50) containing paramagnetic particles (52), said probe (150) comprising :
- a magnet (152),
- a displacement system (154) intended to be arranged at the orifice (106) and constructed to displace the magnet (152) from a capturing position in which the magnet (152) is bathed in the fluid (50) to a checking position in which the magnet (152) is outside of the tank (102), wherein the displacement system (154) is arranged to displace the magnet (152) through the orifice (106) from the inside to the outside of the tank (102) and vice versa, and
- sealing means (170) intended to ensure the seal-tightness of the orifice (106) in the transition from the capturing position to the checking position and vice versa, the probe (150) being **characterized in that** the displacement system (154) takes the form of a screw with a threaded rod (154a) and a head (154b), **in that** the orifice (106) is a tapped hole into which the threaded rod (154a) is screwed, **in that** the threaded rod (154a) has a recess (156) in which the magnet (152) is fixed and **in that** the position of the recess (156) along the threaded rod (154a) is such that the magnet (152) is bathed in the fluid (50) in capturing position and is outside of the tank (102) in checking position.

2. Probe (150) according Claim 1, **characterized in that** the sealing means (170) are produced by the threading of the threaded rod (154a) and the tapping of the orifice (106).

3. Probe (150) according to either Claim 1 or 2, **characterized in that** the screw is produced in a non-magnetic material.

4. Probe (150) according to one of the Claims 1 to 3, **characterized in that** the recess (156) takes the form of at least one tunnel (175) emerging at each end at the threading of the threaded rod (154a), **in that** the or each tunnel (175) has a drain (178) extending along said tunnel (175), **in that** the magnet (152) is housed in the drain (178) at a distance from the threading, and **in that**, for each part of a drain (178) extending between the magnet (152) and the threading of the threaded rod (154a), an element (176) produced in a non-magnetic material is housed in said part.

5. Assembly (100) comprising a tank (102) having a wall (104) pierced by an orifice (106) and in which the tank (102) is intended to contain a fluid (50) containing paramagnetic particles (52) and a probe (150) according to one of the preceding claims, in which the displacement system (154) is arranged at the orifice (106) and arranged to displace the magnet (152) from a capturing position in which the magnet (152) is bathed in the fluid (50) to a checking position in which the magnet (152) is outside of the tank (102), and in which the sealing means (170) are arranged to ensure the seal-tightness of the orifice (106) in the transition from the capturing position to the checking position and vice versa.
